# EUROPEAN PATENT APPLICATION

(11) **EP 4 008 286 A1**
(43) Date of publication of application: **08.06.2022**
(21) Application number: 21212193.3
(22) Date of filing: 03.12.2021
(51) Int. Cl.: A61B 34/20

(54) **INSERTION DEVICE POSITIONING GUIDANCE SYSTEM**

(30) Priority: 07.12.2020 US 202063122046 P
(71) Applicant: Envizion Medical Ltd., 6971060 Tel Aviv (IL)
(72) Inventor: BESSER, Doron, 6935927 Tel Aviv (IL); BEN EZRA, Guy, 3707992 Karkur (IL); COHEN, Ran, 4972626 Petah-Tikva (IL); HILLMAN, Ayelet, 3824261 Hadera (IL); HOFSHI, Anat, 6274403 Tel Aviv (IL)
(74) Representative: AWA Denmark A/S

(57) **Abstract**

There is provided herein a device, system and a method for guiding insertion of an insertion tube into a patient implanted with an implantable device capable of interfering with an electromagnetic field generated over the torso of the patient, the guiding including receiving signals relating to changes in the electromagnetic field, the changes caused by insertion of a gastro-enteral tube including an electromagnetic sensor; normalizing the received signals based on the information regarding the implantable device and its interference with the electromagnetic field, and determining the position of the gastro-enteral tube based on the normalized signal.

## Description

### FIELD OF INVENTION

Embodiments of the disclosure relate to device, system and method for guiding tube positioning, specifically device, system and method for guiding tube positioning based on sensing of changes in an electromagnetic field caused while taking into account disturbances to the electromagnetic fields caused by implantable devices..

### BACKGROUND

Enteral feeding is often used as nutritional support in patients unable to be fed otherwise. Although many benefits are associated with early initiation of enteral feeding, misplacement of feeding tubes is relatively common and can result in patient discomfort and complications. Confirming the position of the tube only after it is already inserted delays the feeding and the initiating of hydration or medication. Bedside electromagnetic (EM) systems for guided placement of nasoenteral feeding tubes are available and are utilized by medical staff during the procedure to avoid misplacement of feeding tubes.

Guiding of tube insertion may be particularly complicated in patient's implanted with implantable devices of various kinds, such as ventricular assist devices, which may cause disturbances to the electromagnetic fields used during the guided insertion.

There is therefore a need, however, for reliable real-time electromagnetic-based tracking systems that provide enhanced accuracy for critical tool positioning during medical procedures, while taking into account disturbances to the electromagnetic fields caused by an implanted device.

### SUMMARY

One of the problems often associated with an insertion of a feeding tube using an electromagnetic positioning guidance system, is that reliability is difficult to obtain in the patient environment, which is typically dynamic. This problem is further enhanced when the patient is implanted with an implantable device the operation of which cause disturbances to the electromagnetic field utilized for tracking the insertion.

According to some embodiments, there is provided a tube positioning device comprising: a processing circuitry configured to track insertion of an insertion tube (e.g. a feeding tube) based on changes in an electromagnetic field generated over the patient's body, the changes caused by an electromagnetic sensor positioned on the tube, while discarding changes caused by operation of a medical device implanted into the patient. Accordingly, the herein disclosed device may advantageously provide reliable and efficient guiding of the insertion in patient implanted with a medical device that may otherwise impair guiding.

According to some embodiments, the processing circuitry is configured to receive information regarding an implantable device of a patient, the implantable device capable of interfering with an electromagnetic field generated over the torso of the patient; receive signals relating to changes in the electromagnetic field, the changes caused by insertion of a gastro-enteral tube comprising an electromagnetic sensor; normalize the received signals based on the information regarding the implantable device and its interference with the electromagnetic field; and determine the position of the gastro-enteral tube, based on the normalized signal.

According to some embodiments, the implantable device is a ventricular assist device.

According to some embodiments, the receiving of the information regarding an implantable device comprises measuring changes in the electromagnetic field caused by the implantable device, in absence of the gastro-enteral tube, as compared to a baseline electromagnetic field measured in absence of the implantable device.

According to some embodiments, the the measuring of the changes in the electromagnetic field caused by the implantable device comprises identifying one or more characteristics of the interference.

According to some embodiments, the implantable device is a periodically operating device.

According to some embodiments, the processing circuitry is configured to identify operation of the implantable device, based on the one or more characteristics of the interference.

According to some embodiments, the receiving of the information regarding an implantable device comprises receiving information regarding the type of the implantable device, wherein the interference of the implantable device with the electromagnetic field is assessed based on the type of the implantable device.

According to some embodiments, there is provided a tube positioning device comprising a processing circuitry configured to receive signals relating to changes in an electromagnetic field generated over the torso of a patient, the changes caused by insertion of a gastro-enteral tube comprising an electromagnetic sensor; normalize the received signals, based on interferences with the electromagnetic field, characteristic to presence of an implantable device; and determine the position of the gastro-enteral tube based on the normalized signal.

According to some embodiments, the implantable device is a ventricular assist device.

According to some embodiments, there is provided a gastroenteral tube positioning system comprising: an electromagnetic field generator configured to generate an electromagnetic field covering a treatment area; wherein said electromagnetic field generator is external to the patient; a registration sensor configured to mark one or more anatomic locations on the patient's torso; a gastroenteral tube comprising an electromagnetic sensor, the electromagnetic sensor configured to cause changes in the electromagnetic field; and a processing circuitry.

According to some embodiments, the processing circuit is configured to generate an anatomical map of the subject's torso or match a subject's torso to a predefined anatomical map, based on the at least one anatomic location marked by the registration sensor, receive signals relating to changes in the electromagnetic field, the changes caused by insertion of the gastro-enteral tube; normalize the received signals, based on the information regarding to a device implanted into the subject and the interference of the implantable device with the electromagnetic field; and show on the map a path of the gastroenteral tube insertion; based on changes in the normalized signal.

According to some embodiments, the processing circuit is configured to receive information regarding the implantable device of a patient.

According to some embodiments, the receiving of the information regarding an implantable device comprises measuring changes in the electromagnetic field caused by the implantable device, in absence of the gastro-enteral tube, as compared to a baseline electromagnetic field measured in absence of the implantable device.

According to some embodiments, the measuring of the changes in the electromagnetic field caused by the implantable device comprises identifying one or more characteristics of the interference.

According to some embodiments, the implantable device is a periodically operating device.

According to some embodiments, the processing circuitry is configured to identify operation of the implantable device based on the one or more characteristics of the interference.

According to some embodiments, the receiving of the information regarding an implantable device comprises receiving information regarding the type of the implantable device, wherein the interference of the implantable device with the electromagnetic field is assessed based on the type of the implantable device.

According to some embodiments, the one or more anatomic locations comprises the suprasternal notch and/or the xiphoid process.

According to some embodiments, the implantable device is a ventricular assist device.

According to some embodiments, there is provided a method for guiding insertion of a gastroenteral tube into a subject, the method comprising applying an electromagnetic field covering a torso of the subject; utilizing an external electromagnetic field generator, marking at least one anatomic location on the subject's torso, utilizing a registration sensor; inserting into the subject a gastroenteral tube comprising an electromagnetic sensor, the electromagnetic sensor configured to cause changes in the electromagnetic field; and utilizing a processing circuitry to: generate an anatomical map of the subject's torso or match a subject's torso to a predefined anatomical map, based on the at least one anatomic location marked by the registration sensor, receive signals relating to changes in the electromagnetic field, the changes caused by insertion of the gastro-enteral tube; normalize the received signals, based on the information regarding to a device implanted into the subject and the interference of the implantable device with the electromagnetic field; and show on the map a path of the gastroenteral tube insertion; based on changes in the normalized signal.

The method of claim 22, wherein the processing circuit is further configured to receive information regarding the implantable device of a patient.

According to some embodiments, the receiving of the information regarding an implantable device comprises measuring changes in the electromagnetic field caused by the implantable device, in absence of the gastro-enteral tube, as compared to a baseline electromagnetic field measured in absence of the implantable device.

According to some embodiments, the measuring of the changes in the electromagnetic field caused by the implantable device comprises identifying one or more characteristics of the interference.

According to some embodiments, the implantable device is a periodically operating device.

According to some embodiments, the processing circuitry is configured to identify operation of the implantable device based on the one or more characteristics of the interference.

According to some embodiments, the receiving of the information regarding an implantable device comprises receiving information regarding the type of the implantable device, wherein the interference of the implantable device with the electromagnetic field is assessed based on the type of the implantable device.

According to some embodiments, the one or more anatomic locations comprises the suprasternal notch and/or the xiphoid process.

According to some embodiments, the implantable device is a ventricular assist device.

Certain embodiments of the present disclosure may include some, all, or none of the above advantages. One or more technical advantages may be readily apparent to those skilled in the art from the figures, descriptions and claims included herein. Moreover, while specific advantages have been enumerated above, various embodiments may include all, some or none of the enumerated advantages.

### BRIEF DESCRIPTION OF THE FIGURES

Some embodiments of the disclosure are described herein with reference to the accompanying figures. The description, together with the figures, makes apparent to a person having ordinary skill in the art how some embodiments of the disclosure may be practiced. The figures are for the purpose of illustrative discussion and no attempt is made to show details of an embodiment in more detail than is necessary for a fundamental understanding of the teachings of the disclosure.
**FIG. 1** is a block diagram of an insertion device positioning guidance system, according to some embodiments;
**FIG. 2** is a flowchart of a method for guiding insertion of an insertion tube in a patient implanted with an implantable device, according to some embodiments;
**FIG. 3** is a flowchart suitable for use in guiding insertion of an insertion tube in a patient implanted with an implantable device, according to some embodiments.

### DETAILED DESCRIPTION

In the following description, various aspects of the disclosure will be described. For the purpose of explanation, specific configurations and details are set forth in order to provide a thorough understanding of the different aspects of the disclosure. However, it will also be apparent to one skilled in the art that the disclosure may be practiced without specific details being presented herein. Furthermore, well-known features may be omitted or simplified in order not to obscure the disclosure.

Disclosed herein is a system and method for guiding insertion of an insertable medical device (e.g., a tube, such as a feeding tube). The disclosed system may be used as an insertion device positioning guidance system. The system may be used to track and indicate, in real time, the location of an insertion medical device during the insertion process. As one example, the system may track and indicate the location of a tip of a feeding tube as it is being inserted into the body of a subject, while taking into account devices 9such as implantable devices) interfering with or causing disturbances to the guiding. Advantageously, this makes the insertion procedure considerably easier and safer, ensuring that the tube is inserted at a correct location.

According to some embodiments, there is provided an insertion device positioning guidance system comprising: an electromagnetic field generator, configured to generate an electromagnetic field covering a treatment area; a reference sensor configured to be positioned, within the treatment area, on the subject's torso, the reference sensor is configured to define a reference coordinate system representing the position and orientation of the subject's torso relative to said field generator; a registration sensor configured to mark at least four anatomic locations relative to the reference coordinate system; and processing circuitry configured to operate said field generator, read signals obtained from said reference sensor and said registration sensor, calculate a position and orientation thereof relative to said field generator, generate an anatomical map representing the torso of the subject and at least a first and a second of the at least four anatomic locations, said processor/processing circuitry is further configured to facilitate visualization on the anatomical map of a position, orientation and path of a tip sensor, located in a distal tip section of the insertion device, with respect to the first and second anatomic locations, independent of the subject's movement and independent of deviations in the position and/or orientation of said field generator, thus determination of a successful medical procedure is facilitated. Optionally, the system further includes a monitor configured to display the map.

A reference coordinate system representing the position and orientation of the subject's torso relative to the field generator may be indicated by a reference sensor configured to be positioned, within the treatment area, on a subject's torso. The reference sensor may be positioned on a side of the patient's torso, such that the anatomical map further depicts a body contour of the subject.

The first and the second anatomic locations may be indicated by a registration sensor configured to mark at least the first and the second anatomic locations relative to the reference coordinate system. Optionally, the registration sensor is a stylus configured to be manually operated. Optionally, the first anatomic location is the suprasternal notch and the second anatomic location is the xiphoid process, and a path display of the gastroenteral tube relative to the first and second anatomic locations is indicative of a successful insertion.

According to some embodiments, the first and/or second anatomic location may indirectly derived based on other anatomic locations marked by the registration sensor, According to some embodiments, the other anatomic locations may be the left and right claviculae. According to some embodiments, the left and right claviculae along with the xiphoid process may be used for calculating a position, orientation and/or posture of the subject. According to some embodiments, calculating a position, orientation and/or posture of the subject may include determining/calculating/drawing within the coordinate system, a first vector (v1) extending between the xiphoid process and the left clavicula and a second vector (v2) extending between the xiphoid process and the right clavicula, based on the registration of the xiphoid process and the left and right claviculae by the registration sensor. According to some embodiments, the processing circuitry may be configured to determine/calculate/draw a third vector (v3) based on the first and second vectors (v1 and v2), for example by calculating the cross product of vl with v2, the third vector being indicative of the position, orientation and/or posture of the subject.

According to some embodiments, the electromagnetic field generator may be external to the patient. According to some embodiments, the electromagnetic field generator may be static throughout a duration of a procedure for placing a tube within a body of a subject. In such cases, a region covered by the electromagnetic field is static/constant throughout the duration of a procedure for placing a tube within a body of a subject. Advantageously, the static electromagnetic field may contribute to the accuracy of the display.

The anatomical map may show a frontal upper view of the subject and/or a side view of the subject and/or an axial view of the subject.

One example of hardware suitable for use as the abovementioned electromagnetic tracking system, including the electromagnetic field generator and one or more of the sensors, is the Aurora^{®} system by Northern Digital Inc., of Ontario, Canada.

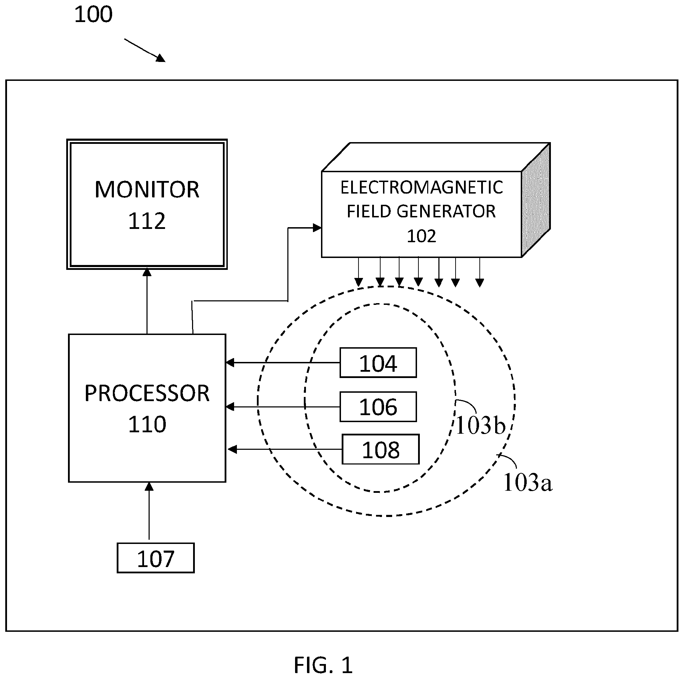

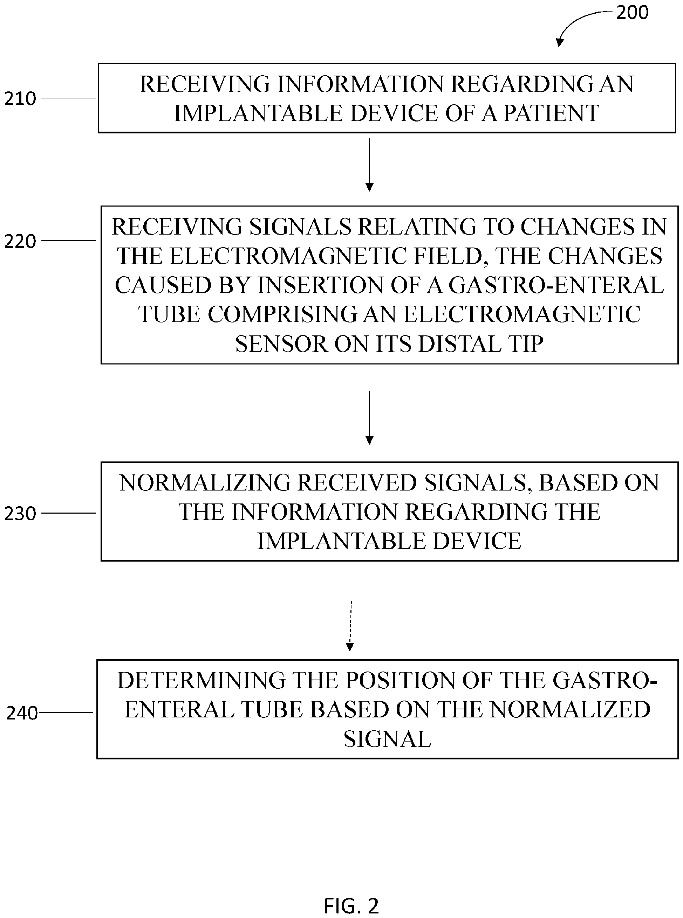

Throughout the following description, similar elements of different embodiments of the device are referenced by element numbers differing by integer multiples of 100. For example, an electromagnetic field generator of **Fig. 1** is referenced by the number **102**, and an electromagnetic field generator of **Fig. 2**, which corresponds to electromagnetic field generator **102** of **Fig. 1**, is referenced by the number **202.**

Reference is now made to **FIG. 1** which is a block diagram of an insertion device positioning guidance system **100.** System **100** includes an electromagnetic field generator **102** configured to generate an electromagnetic field **103a** covering at least a region of interest **103b** (e.g., a treatment area such as a patient's torso), an electromagnetic sensor, such as sensors **104** located in/on a distal tip section of the insertion tube of the region of interest **103b** (typically the subject's torso). System **100** further includes a processor **110** configured to operate the field generator, read signals obtained from sensor **104**, and to display on a monitor **112** the position and/or path of the insertion tube on an anatomical map representing the torso of the subject. independently of the subject's movement, independent of deviations in the position and/or orientation of field generator **102,** while correcting for deviations caused by presence of an implantable device **106.**

In some embodiments, monitor **112** may be integrated with processor **110**, such as in the case of an all-in-one computer.

According to some embodiments, processor **110** is configured to receive information regarding the presence of implantable device **106** and/or type of implantable device **106** e.g. by receiving a signal from the implantable device, by detection of the medical device and/or by obtaining an input for example from a user interface. According to some embodiments, the calculating of the position and/or path of the insertion tube is based on the information received regarding the implantable device. Additionally or alternatively, the processing unit may be configured to discard and/or normalize the signals obtained from sensor **104** irrespective of presence/absence of an implantable device.

According to some embodiments, the system further includes a reference sensor **108** configured to be positioned on a subject's torso. Reference sensor **108** is configured to define a reference coordinate system representing the position and orientation of the subject's torso relative to the field generator. Optionally, reference sensor **108** may be attached to the skin of the patient, for example on the side of a patient's torso such as beneath the patient's armpit. In such cases, the anatomical map further depicts a body contour of the subject. Reference sensor **108** may be, for example, a 6-DOF electromagnetic sensor, capable of determining 6 axes of its location (XYZ axes) and attitude (roll, yaw, and pitch) with respect to field generator **102.**

According to some embodiments, the system further includes a registration sensor **107** configured to be positioned on and/or to mark anatomic (thoracic) locations over the subject's body (e.g. the subject's torso). Different anatomical locations may be marked depending on the type of procedure used, the type of insertion medical device, etc. The marking of the anatomic location may be physical, such as attaching a marker/fiducial (such as a sticker). Alternatively, the marking of the anatomic location may be virtual, such as registering a virtual marker/fiducial. The marking, in accordance with embodiments, may facilitate identification or designation of an anatomical location within or on a subject's body such as, in a non-limiting example, a subject's suprasternal notch, and a subject's xiphoid process.

Optionally, registration sensor **107** is a stylus sensor having a 3 DOF sensor on its distal tip, the stylus configured to be manually operated to mark at least three anatomic location over the subject's body identified by the operator of the stylus. The marking may be made, merely as an example, by indicating to the software (for example, but not limited to) by pressing a GUI button or voice activation) once stylus sensor **107** is positioned over the desired point on the patient's body. The marking may be communicated to and registered by processor **110.**

According to some embodiments, as used herein the terms "insertion device", "insertion medical device" and "insertion tube" may be used interchangeably and may refer to any device/tool adapted for insertion into a body. The insertion device may be any medical insertion device or a medical surgical device. Non-limiting examples of insertion medical devices include, feeding tubes, such as gastroenteral tubes (for example, nasoenteral feeding tubes), endotracheal tube, tracheostomy tube, stomach tube, catheter tubes or cricothyrotomy tube. Other examples of insertion devices are well known in the art.

According to some embodiments, the terms "processing circuitry" and "processor" may be used interchangeably.

In some embodiments, the insertion device is a tube. In some embodiments, the tube is a feeding tube. In some embodiments, the tube is a gastro/enteral feeding tube, such as, but not limited to, a nasogastric feeding tube or a naso-enteral feeding tube. According to some embodiments, the feeding tube may have disposed therein and/or thereon an electromagnetic sensor, for example at its distal end.

Reference is now made to **FIG. 2** which is a flow chart of a method **200** for guiding insertion of an insertion tube, according to some embodiments.

It is understood to one of ordinary skill in the art that the order of at least of forthcoming steps of the method may be changed and/or be performed in parallel.

According to some embodiments, the method is implemented by a processing unit or a non-transitory computer readable medium having stored thereon software instructions that, when executed by a processor, cause the processor to perform the herein below described steps.

In step **210** information regarding an implantable device of a patient capable of interfering and/or causing disturbances to an electromagnetic field generated over the torso of the patient is received. According to some embodiments, receiving the information may include receiving it as an input indication (e.g. via a user interface), receiving a signal from the implantable device and/or deriving the presence and/or type of implantable device based on one or more signature parameters, the signature parameters optionally related to the type of interference/disturbance caused to the electromagnetic field. According to some embodiments, the information regarding the implantable device may regard the type of the implantable device. According to some embodiments, the information regarding the implantable device may regard the type, degree or patter of the disturbance caused by the implantable device to the electromagnetic field. According to some embodiments, the implantable device is a periodically operating device.

In step **220** signals relating to changes in the electromagnetic field are received. According to some embodiments, at least the majority of the changes to the electromagnetic field are caused by insertion of an insertion tube (e.g. a gastro-enteral tube) into the patient's gastro-enteral system, the insertion tube characterized by including an electromagnetic sensor, e.g. on a distal tip thereof.

In step **230** the received signals are normalized, based on the information regarding the implantable device. and its interference with the electromagnetic field. According to some embodiments, the normalization includes a step of identifying the strength, and/or pattern of the disturbance/interference (also referred to herein as the signature of the of the disturbance/interference). According to some embodiments, the identifying of the signature of the disturbance/interference may include applying pattern recognition algorithms. According to some embodiments, the normalization includes deducing the changes in the electromagnetic filed caused by the implantable device. According to some embodiments, the identification and/or normalization includes the applying of machine-learning algorithms. According to some embodiments, the normalization eliminates the disturbances. According to some embodiments, the normalization reduces the disturbances. According to some embodiments, the normalization includes a step of identifying operation of the implantable device based on the one or more characteristics of the interference.

In step **240** the position of the gastro-enteral tube is determined based on the normalized signal.

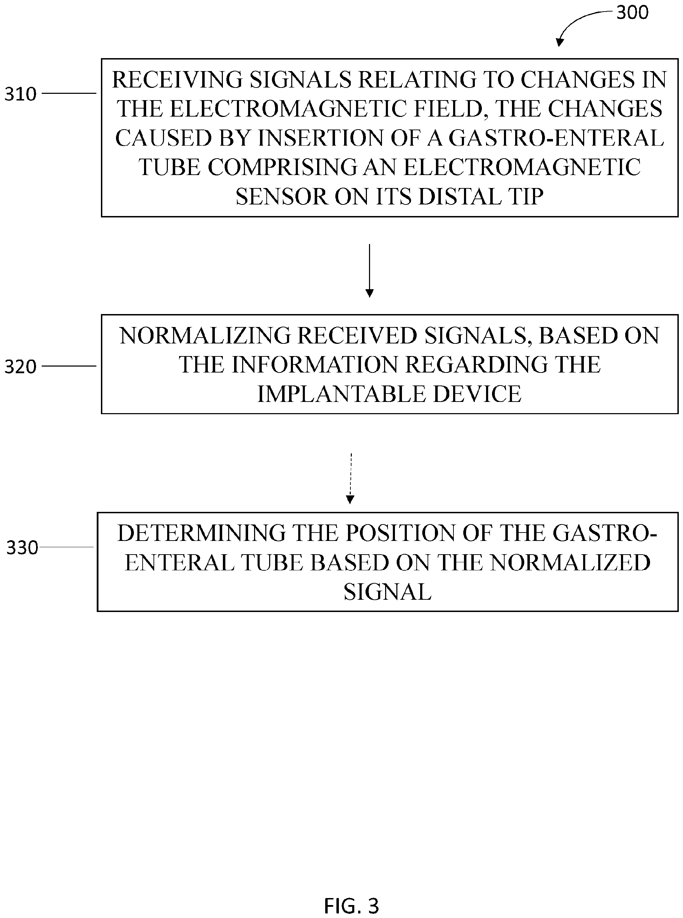

Reference is now made to **FIG. 3** which is a flow chart of a method **300** for guiding insertion of an insertion tube, according to some embodiments.

It is understood to one of ordinary skill in the art that the order of at least of forthcoming steps of the method may be changed and/or be performed in parallel.

According to some embodiments, the method is implemented by a processing unit or a non-transitory computer readable medium having stored thereon software instructions that, when executed by a processor, cause the processor to perform the herein below described steps.

In step **310** signals relating to changes in the electromagnetic field are received. According to some embodiments, at least the majority of the changes to the electromagnetic field are caused by insertion of an insertion tube (e.g. a gastro-enteral tube) into the patient's gastro-enteral system, the insertion tube characterized by including an electromagnetic sensor, e.g. on a distal tip thereof.

In step **320** the received signals are normalized, based on the information disturbances/interferences typically caused if an implantable device in present. According to some embodiments, the identification and/or normalization includes the applying of machine-learning algorithms. According to some embodiments, the normalization eliminates disturbances of the implantable device, if present. According to some embodiments, the normalization reduces the disturbances of the implantable device, if present. According to some embodiments, the normalization minimally affects the signal if no implantable device is present.

In step **330** the position of the gastro-enteral tube is determined based on the normalized signal.

The present invention may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire. Rather, the computer readable storage medium is a non-transient (i.e., not-volatile) medium.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Java, Smalltalk, C++ or the like, and conventional procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

The descriptions of the various embodiments of the present invention have been presented for purposes of illustration, but are not intended to be exhaustive or limited to the embodiments disclosed. Many modifications and variations will be apparent to those of ordinary skill in the art without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

While a number of exemplary aspects and embodiments have been discussed above, those of skill in the art will recognize certain modifications, additions and sub-combinations thereof. It is therefore intended that the following appended claims and claims hereafter introduced be interpreted to include all such modifications, additions and sub-combinations as are within their true spirit and scope.

## Claims

1. A tube positioning device comprising:
a processing circuitry configured to:
receive information regarding an implantable device of a patient, the implantable device capable of interfering with an electromagnetic field generated over the torso of the patient;
receive signals relating to changes in the electromagnetic field, the changes caused by insertion of an insertion tube comprising an electromagnetic sensor;
normalize the received signals based on the information regarding the implantable device and its interference with the electromagnetic field; and
determine the position of the insertion tube based on the normalized signal.

2. The tube positioning device of claim 1, wherein the implantable device is a ventricular assist device.

3. The tube positioning device of claim 1, wherein the receiving of the information regarding an implantable device comprises measuring changes in the electromagnetic field caused by the implantable device, in absence of the insertion tube, as compared to a baseline electromagnetic field measured in absence of the implantable device.

4. The tube positioning device of claim 3, wherein the measuring of the changes in the electromagnetic field caused by the implantable device comprises identifying one or more characteristics of the interference.

5. The tube positioning device of any one of claims 4, wherein the implantable device is a periodically operating device.

6. The tube positioning device of claim 4, wherein the processing circuitry is configured to identify operation of the implantable device based on the one or more characteristics of the interference.

7. The tube positioning device of claim 1, wherein the receiving of the information regarding an implantable device comprises receiving information regarding the type of the implantable device, wherein the interference of the implantable device with the electromagnetic field is assessed based on the type of the implantable device.

8. An insertion tube positioning system comprising:
an electromagnetic field generator configured to generate an electromagnetic field covering a treatment area; wherein said electromagnetic field generator is external to the patient;
a registration sensor configured to mark one or more anatomic locations on the patient's torso;
an tube comprising an electromagnetic sensor, the electromagnetic sensor configured to cause changes in the electromagnetic field; and
a processing circuitry configured to:
generate an anatomical map of the subject's torso or match a subject's torso to a predefined anatomical map, based on the at least one anatomic location marked by the registration sensor,
receive signals relating to changes in the electromagnetic field, the changes caused by insertion of the insertion tube;
normalize the received signals, based on the information regarding to a device implanted into the subject and the interference of the implantable device with the electromagnetic field; and
show on the map a path of the insertion tube insertion; based on changes in the normalized signal.

9. The system of claim 8, wherein the processing circuit is configured to receive information regarding the implantable device of a patient.

10. The system of claim 9 wherein the receiving of the information regarding an implantable device comprises measuring changes in the electromagnetic field caused by the implantable device, in absence of the insertion tube, as compared to a baseline electromagnetic field measured in absence of the implantable device.

11. The system of claim 10, wherein the measuring of the changes in the electromagnetic field caused by the implantable device comprises identifying one or more characteristics of the interference.

12. The system of claim 8, wherein the implantable device is a periodically operating device and wherein the processing circuitry is configured to identify operation of the implantable device based on the one or more characteristics of the interference.

13. The system of claim 9, wherein the receiving of the information regarding an implantable device comprises receiving information regarding the type of the implantable device, wherein the interference of the implantable device with the electromagnetic field is assessed based on the type of the implantable device.

14. The system of any one of claims 8-13, wherein the one or more anatomic locations comprises the suprasternal notch and/or the xiphoid process.

15. The system any one of claims 8-14, wherein the implantable device is a ventricular assist device.
